# EUROPEAN PATENT APPLICATION

(11) **EP 3 318 568 A1**
(43) Date of publication of application: **09.05.2018**
(21) Application number: 16382510.2
(22) Date of filing: 04.11.2016
(51) Int. Cl.: C07D 513/04

(54) **PREPARATION PROCESS OF EDOXABAN TOSYLATE MONOHYDRATE**

(71) Applicant: Esteve Química, S.A., 08024 Barcelona (ES)
(72) Inventor: Berenguer Maimó, Ramón, 08024 Barcelona (ES); Racamonde Villanueva, Marta, 08024 Barcelona (ES); Winter, Stephen Benedict David, 08024 Barcelona (ES)
(74) Representative: ZBM Patents - Zea, Barlocci & Markvardsen

(57) **Abstract**

A preparation process of edoxaban tosylate monohydrate comprising dissolving edoxaban free base, p-toluensulfonic acid in an appropriate amount of ACN/H₂O and in an appropriate volume ratio, followed by crystallizing the product from ACN/H₂O in a different ratio. The process may further comprise an additional purification step comprising the recrystallization of the product in an appropriate amount of ACN/H₂O.

## Description

The present invention relates to a process for preparing edoxaban tosylate monohydrate with high purity and easily industrializable.

### Background art

Edoxaban tosylate monohydrate is the International Non-Proprietary name (INN) of the chemical compound N'-(5-chloropyridin-2-yl)-N²-((1S,2R,4S)-4-[(dimethylamino) carbonyl]-2-{[(5-methyl-4,5,6,7-tetrahydrothiazolo[5,4-c]pyridin-2-yl)carbonyl]amino} cyclohexyl)ethanediamide p-toluenesulfonate monohydrate. The CAS number is 1229194-11-9. The chemical structure of the edoxaban tosylate monohydrate is included below:

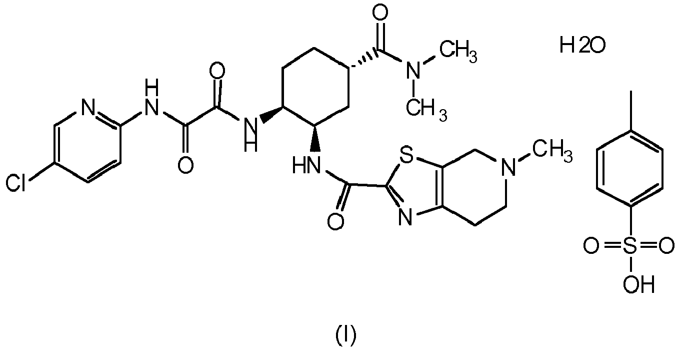

Edoxaban tosylate monohydrate is known as a compound that exhibits an inhibitory effect on activated blood coagulation factor X (FXa) and is useful as a preventive and/or therapeutic drug for thrombotic diseases.

Some methods for its preparation are known in the art which are based on mixing the free form of edoxaban with p-tolunesulfonic acid or p-toluensulfonic acid monohydrate in ethanol/water as solvent system, followed by crystallization of the product from the reaction mixture.

Thus, Example 45 of EP1405852 B1 discloses the preparation of edoxaban tosylate monohydrate from the edoxaban free base dissolved in methylene chloride and p-toluensulfonic acid dissolved in ethanol. The solvent system is distilled off to change the solvent to ethanol/water and the product is crystallized from this mixture. Reference Example 3 of US8686189B2 discloses the preparation of edoxaban tosylate monohydrate by reacting the edoxaban free base and p-toluensulfonic acid monohydrate in 30% hydrous ethanol. The product is crystallized from the reaction mixture after adding more ethanol. Test Example 3 of EP2371830B1 discloses a preparation process of edoxaban tosylate monohydrate in ethanol/water with stable high yields based on the dissolution at a certain temperature of the edoxaban free base and an amount of p-toluensulfonic acid monohydrate below the stoichiometric amount, and adding an excess of p-toluensulfonic acid under low temperature. None of these documents disclose the purity obtained of the edoxaban tosylate monohydrate thus obtained.

WO2015129603A1 discloses a method of purification of edoxaban tosylate monohydrate from some process impurities using a mixture of ethanol/water based on dissolving the compound (I) in a mixture of aqueous ethanol 70%v/v and adding more ethanol at a low temperature to crystallize the product. According to this document, the maximum content of any type of impurity following the purification method disclosed therein is 0.03%a/a and the total content of all impurities is 0.13% a/a or less by HPLC (detection wavelength 290 nm) with respect to the HPLC area value of a free form of the edoxaban. Taking into account that all the Examples were carried out from a starting material having a purity of 99.84% with all the impurities being in the amount of 0.03% a/a or less by HPLC and the total content of all impurities being 0.16% a/a or less by HPLC, and that the products obtained in Examples 1-3 have 99.87-99.88% a/a, the effect on the purification is not significant. Besides, this recrystallization process uses a huge amount of ethanol with respect to the amount of the starting material (21.6 volumes ethanol per weight starting material and 2.4 volumes water per weight of starting material) which is a drawback in terms of process economy.

Finally, the formation of sulfonic salts in the presence of alcoholic solvents as in the processes disclosed above, presents the risk of generating byproduct esters that are alkylating agents, especially if there is a prolonged exposure of the acids to an alcohol at high temperature. The resulting alkyl sulfonates are described as genotoxic as they are direct-acting bacterial mutagens and are almost universally regarded by regulatory authorities to be potential impurities in drug substances presented as sulfonic acid salts, particularly if synthesized using an alcoholic solvent. Therefore, as genotoxic impurities pose a significant safety risk, even at low concentrations, regulatory authorities have specifically defined their limits in drug substances and products.

Therefore, it is desirable to find an efficient, economical and scalable preparation process of edoxaban tosylate monohydrate, which proceeds with high yields and high purity and which also avoids the risk of formation of genotoxic impurities due to the combination of ethanol and p-toluensulfonic acid.

### Summary of the invention

Inventors have found that the behavior of the edoxaban tosylate in a mixture of acetonitrile/water (ACN/H₂O) is unexpected because of both, its solubility properties and its purification properties. Thus, inventors have developed a process for preparing edoxaban tosylate monohydrate using acetonitrile/water as a solvent and which allow obtaining a high purification of the crystallized product. It avoids the risk of formation of genotoxic impurities due to the combination of ethanol and p-toluensulfonic acid, and is advantageous in terms of process economy and subsequent treatment of mother liquors because of the fact that water is the major solvent employed and also the low amount of volumes of solvent used.

Edoxaban tosylate monohydrate is slightly soluble in water. According to the solubility tests carried out by the present inventors, the solubility of edoxaban tosylate monohydrate in water is 2.7 g/kg of solution at room temperature and in acetonitrile it is 0.56 g/Kg of solution at room temperature. However, when a mixture of both acetonitrile and water is used in a certain ratio, the solubility of the edoxaban tosylate monohydrate dramatically increased. Thus, the solubility of edoxaban tosylate monohydrate in a mixture ACN/water 1:1 is 100.2 g/Kg of solution at room temperature, much higher than in ethanol/water in the same ratio (solubility in ethanol at room temperature; 1.29 g/kg of solution; solubility in a mixture of ethanol/water 1:1 at room temperature: 23.9 g/Kg of solution).

Because of the low active pharmaceutical ingredient solubility the choice of solvent may be critical. Ethanol and acetonitrile belong to different classes of solvents, ethanol is a polar protic solvent and acetonitrile is a polar aprotic solvent. However, this increased solubility behavior of the edoxaban tosylate monohydrate is not observed in other polar aprotic solvents. Thus, edoxaban tosylate monohydrate solubility in a mixture of acetone/water 1:1 at room temperature is 33 g/kg of solution and in a mixture of dioxane/water 1:1 at room temperature is 31 g/kg of solution.

On the other hand, all the processes of the prior art encompass a large increase of the ratio of ethanol in the mixture ethanol/water in order to crystallize the edoxaban tosylate monohydrate, being the final volume of solvents too high in terms of process economy. Conversely, in the process of the present invention the major solvent for the crystallization is water and the total amounts of solvents are quite lower than the ones of the processes of the prior art. Thus, the process is cleaner and more economical with respect to the known processes. In addition, the yield of the process is high, approximately of the same order than the ones of the prior art.

Thus, the behavior in terms of solubility of the edoxaban tosylate monohydrate in a mixture of ACN/H₂O with respect to the single solvents and with respect to other aqueous solvents, as well as the specific conditions of the process, allows both the use of a ratio of solvents that allows dissolving the product in small volumes, and the use of another ratio of them, where water is the major solvent, that allows crystallizing the product with high yields and without adversely affecting to the purification of the product, in fact, the purification of some impurities is even better than the one of the processes of the prior art.

Therefore, an aspect of the present invention relates to the provision of a preparation process of edoxaban tosylate monohydrate comprising the steps of a) combining edoxaban free base, p-toluensulfonic acid in a mixture of ACN/H₂O in a volume ratio of from 70:30 to 30:70 and heating the mixture until reaching complete dissolution, wherein the mixture of ACN/H₂O is in such an amount that the edoxaban tosylate formed is dissolved at a temperature of from 30 to 70 °C; b) Cooling the solution thus obtained in a) to a temperature equal to or below than room temperature; c) Adding water up to a final volume ratio of ACN/H₂O of from 10:90 to 30:70; and d) Recovering the edoxaban tosylate monohydrate from the reaction media, wherein steps b) and c) are carried out in any order.

It is also part of the invention a purification process of edoxaban tosylate monohydrate comprising: a) Dissolving edoxaban tosylate monohydrate in a mixture of ACN/H₂O in a volume ratio ACN/H₂O from 60:40 to 30:70 at an appropriate temperature; wherein the mixture of ACN/H₂O is in such an amount that the edoxaban tosylate is dissolved at a temperature of from 30-70 °C; b) Cooling the solution thus obtained in a) up to a temperature equal to or below than room temperature; c) Adding water up to a final volume ratio of ACN/H₂O of from 10:90 to 30:70; and d) Recovering the edoxaban tosylate monohydrate from the crystallization media, wherein steps b) and c) are carried out in any order.

### Brief description of drawings

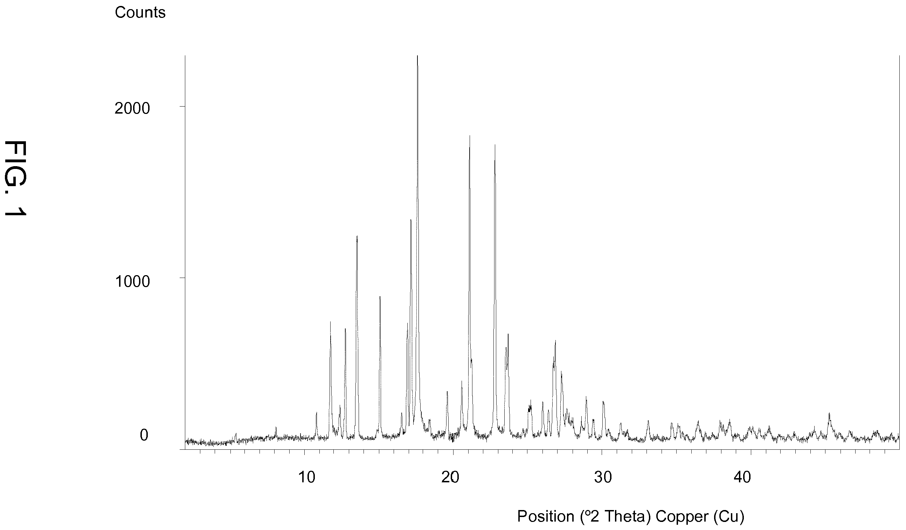

FIG. 1 shows a powder X-ray diffractogram of Form I of edoxaban tosylate monohydrate reference standard used for comparison in the Examples.

### Detailed description of the invention

The term "room temperature" as disclosed herein refers to a temperature of the environment, without heating or cooling, and is generally comprised of from 20 to 25 °C. For the purposes of the invention, any ranges given include both the lower and the upper end-points of the range. Ranges given, such as temperatures, times, and the like, should be considered approximate, unless specifically stated.

The percentages of impurities given refer to the percentage with respect to the HPLC area value of a free form of the edoxaban (%a/a).

As mentioned above the invention relates to a preparation process of edoxaban tosylate monohydrate comprising: a) combining edoxaban free base, p-toluensulfonic acid in a mixture of ACN/H₂O in a volume ratio of from 70:30 to 30:70 and heating the mixture until reaching complete dissolution, wherein the mixture of ACN/H₂O is in such an amount that the edoxaban tosylate formed is dissolved at a temperature of from 30 to 70 °C; b) Cooling the solution thus obtained in a) to a temperature equal to or below than room temperature; c) Adding water up to a final volume ratio of ACN/H₂O of from 10:90 to 30:70; and d) Recovering the edoxaban tosylate monohydrate from the reaction media, wherein steps b) and c) are carried out in any order.

The edoxaban tosylate monohydrate obtained by the process of the present invention has a high purity. In particular a high purification of the following impurities is observed:
a) N1-((1S,2R,4S)-2-amino-4-(dimethylcarbamoyl)cyclohexyl)-N2-(5-chloropyrid-2-yl)oxalamide, impurity A: this impurity is an intermediate for the preparation of edoxaban. 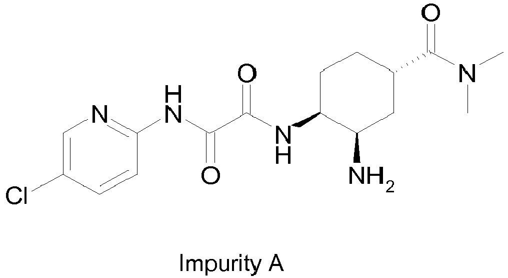
b) Degradation impurities produced during the preparation process of the tosylate salt of edoxaban, in both ethanol/water and acetonitrile/water. 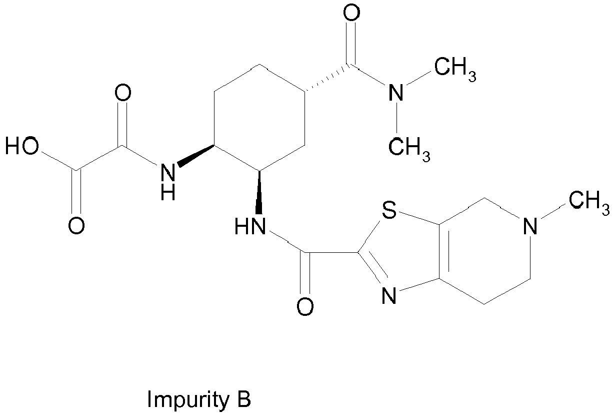 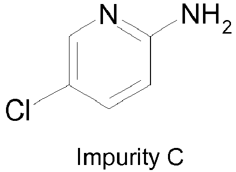
c) Salt of edoxaban with an isomer of the p-toluensulfonic acid (orto isomer).This impurity, named Impurity D, is related to the quality of the p-toluensulfonic acid used.

In the context of the present invention, any of the impurities mentioned above, has a content equal to or lower than 0.1 % a/a, in a particular embodiment, if present, they are in an amount equal to or lower than 0.05% a/a. Preferably, the impurities are absent or not detected (nd) by analytical methods such as HPLC.

The Edoxaban tosylate monohydrate thus obtained corresponds to Form I. Form I has a powder X-ray diffractogram that comprises peaks at approximately 11.8, 12.7, 13.5, 15.1, 16.9, 17.2, 17.6,21.1, 22.8, 23.4, 26.7, 26.8, and 27.3 degrees 2 theta.
In particular, the diffractogram comprises the peaks of Table 1.

**Table 1:**

| **Peak Number** | **Pos. [°2Th.]** | **d-spacing [Å]** | **Height [cts]** | **Rel. Int. [%]** |
|---|---|---|---|---|
| 1 | 5.34 | 16.54 | 34 | 1.5 |
| 2 | 8.09 | 10.91 | 68 | 3.1 |
| 3 | 10.81 | 8.18 | 133 | 6.0 |
| 4 | 11.77 | 7.51 | 674 | 30.3 |
| 5 | 12.40 | 7.13 | 175 | 7.9 |
| 6 | 12.74 | 6.94 | 632 | 28.4 |
| 7 | 13.54 | 6.53 | 1176 | 52.9 |
| 8 | 15.06 | 5.88 | 828 | 37.2 |
| 9 | 16.50 | 5.37 | 102 | 4.6 |
| 10 | 16.93 | 5.23 | 669 | 30.1 |
| 11 | 17.17 | 5.16 | 1267 | 57.0 |
| 12 | 17.60 | 5.04 | 2223 | 100.0 |
| 13 | 18.45 | 4.80 | 100 | 4.5 |
| 14 | 19.62 | 4.52 | 255 | 11.5 |
| 15 | 20.55 | 4.32 | 314 | 14.1 |
| 16 | 21.08 | 4.21 | 1745 | 78.5 |
| 17 | 22.78 | 3.90 | 1696 | 76.3 |
| 18 | 23.56 | 3.77 | 518 | 23.3 |
| 19 | 23.69 | 3.75 | 596 | 26.8 |
| 20 | 25.17 | 3.53 | 180 | 8.1 |
| 21 | 25.97 | 3.43 | 146 | 6.6 |
| 22 | 26.41 | 3.37 | 145 | 6.5 |
| 23 | 26.74 | 3.33 | 454 | 20.4 |
| 24 | 26.85 | 3.32 | 551 | 24.8 |
| 25 | 27.27 | 3.27 | 366 | 16.5 |
| 26 | 27.59 | 3.23 | 123 | 5.5 |
| 27 | 27.97 | 3.19 | 98 | 4.4 |
| 28 | 28.94 | 3.08 | 224 | 10.1 |
| 29 | 29.39 | 3.04 | 102 | 4.6 |
| 30 | 30.08 | 2.97 | 215 | 9.7 |
| 31 | 30.44 | 2.93 | 54 | 2.4 |
| 32 | 31.26 | 2.86 | 100 | 4.5 |
| 33 | 33.10 | 2.70 | 114 | 5.1 |
| 34 | 45.26 | 2.00 | 156 | 7.0 |

The water content determined by Karl Fischer method is around 2.4%, corresponding to a monohydrate.

Generally, the edoxaban tosylate monohydrate obtained by the process of the present invention has a particle size distribution with a d90 equal to or lower than 150 µm as determined by laser diffraction analysis.

The poor aqueous solubility of edoxaban tosylate monohydrate limits *in vivo* bioavailability owing to its low dissolution rate in the gastrointestinal fluids following oral administration. Reduction of particle size is a conventional method to increase solubility, in particular, the micronisation of the product. However, micronisation may result in an active agent with undesirably poor flowability, it makes the handling more difficult and complicated with regard to health and safety, and the considerable enlargement of the surface area during micronisation may also cause the sensitivity of the active agent to oxidation to increase. Besides, micronisation implies also a product loss. Advantageously, the process of the present invention allows obtaining the product with different particle sizes, including small particle sizes as disclosed in the Examples.

In a particular embodiment of the process, a molar ratio of edoxaban free base and p-toluensulfonic acid comprised of from 1:1 to 1:1.1 is used. More preferably, the molar ratio is 1:1.05.

In a particular embodiment of the process, the volume ratio of ACN/H₂O in step a) is of from 60:40 to 30:70. In another particular embodiment, of the process, the volume ratio of ACN/H₂O in step a) is of from 50:50 to 35:65. In another particular embodiment, the volume ratio is 50:50.

In another particular embodiment of the process, the amount of ACN/H₂O in step a) is of from 4 to 7 volumes of the mixture per weight of edoxaban free base starting material. In another particular embodiment of the process, the amount of ACN/H₂O in step a) is of from 4 to 6 volumes of the mixture per weight of edoxaban free base starting material. The acetonitrile and the water can be added separately or as a previous prepared mixture in the volume ratios specified.

Generally, the starting edoxaban tosylate monohydrate is dissolved at a temperature of from 30 to 70 °C, preferably at a temperature of from 65 to 70 °C. Insoluble matter may be separated at such temperature, for instance, by filtration and washing of the filtrate with a small amount of the solvent mixture.

A gradual cooling to room temperature is generally carried out. Optionally, the process may comprise a seeding step to favor the crystallization of the product. The seed crystals are crystals of Form I of edoxaban tosylate monohydrate. Preferably the cooling is continued until reaching a temperature of from 0 to 5 °C.

Generally, the additional water is added at a temperature comprised of from 50 to 0-5 °C. In a particular embodiment, the water is added at room temperature, in another particular embodiment, the water is added at 0-5 °C.

In a particular embodiment, the cooling step is carried out until a temperature of 0-5 °C and the additional water is added at such temperature. In another particular embodiment, the amount of water added in step c) is of from 6 to 11 volumes per weight of edoxaban free base starting material. In another particular embodiment, the amount of water added in step c) is of from 7 to 10 volumes per weight of edoxaban free base starting material.

In another particular embodiment of the process, the volume ratio of ACN/H₂O of the final mixture of ACN/H₂O of step c) is of from 15:85 to 25:75. In another particular embodiment, the volume ratio of ACN/H₂O of the final mixture of ACN/H₂O is 20:80. In another particular embodiment of the process, the total amount of acetonitrile/water in step c) is of from 10-18 volumes of a mixture of ACN/H₂O per weight of edoxaban free base starting material.

The separation of the product from the supernatant solution can be done by a conventional method such as filtration, including washing the collected product with the same solvent system used. Thus, in a preferred embodiment of the process, the recovering in step d) comprises filtering the product thus obtained. The solid filters readily thereby making the preparation easier. The remaining solvent can be removed from the product by drying under vacuum.

The combination of the one or more of the previous embodiments is also part of the invention.

The process of the invention, when appropriate, may also comprise a recrystallization. Thus, for instance, when the contents of any of the impurities mentioned above in the edoxaban tosylate monohydrate obtained by the process of the present invention, are equal to or higher than 0.05% a/a, in particular, equal to or higher than 0.1 % a/a an additional purification step could be carried out. This generally will occur in cases where the starting material to prepare the tosylate salt had a high content of impurities. Thus, in such a case, the process of the present invention may further comprise one or more additional purification steps. In particular, the process may further comprise the steps of: e) Dissolving the edoxaban tosylate monohydrate obtained as disclosed above in a mixture of ACN/H₂O in a volume ratio ACN/H₂O from 60:40 to 30:70 at an appropriate temperature; wherein the mixture of ACN/H₂O is in such an amount that the edoxaban tosylate is dissolved at a temperature of from 30-70 °C; f) Cooling the solution thus obtained in a) up to a temperature equal to or below than room temperature; g) Adding water up to a final volume ratio of ACN/H₂O of from 10:90 to 30:70; and h) Recovering the edoxaban tosylate monohydrate from the crystallization media, wherein steps f) and g) are carried out in any order.

The edoxaban tosylate monohydrate thus obtained has a high purity, in particular, impurity A, impurity B, impurity C and impurity D do not exceed 0.05% a/a. More preferably, they do not exceed 0.03% a/a. Even more preferably, the impurity is absent or not detected (nd) by analytical methods such as HPLC.

Generally, the edoxaban tosylate monohydrate obtained after the recrystallization also has a particle size distribution with a d90 equal to or lower than 150 µm as determined by laser diffraction analysis and corresponds to Form I.

When the process of the invention encompasses the preparation of the edoxaban tosylate monohydrate and a subsequent recrystallization, the edoxaban tosylate monohydrate may be recrystallized without carrying out a drying step of the starting material of the recrystallization.

It is also considered part of the invention, a purification process of edoxaban tosylate monohydrate comprising: (A) Dissolving edoxaban tosylate monohydrate in a mixture of ACN/H₂O in a volume ratio ACN/H₂O from 60:40 to 30:70 at an appropriate temperature; wherein the mixture of ACN/H₂O is in such an amount that the edoxaban tosylate is dissolved at a temperature of from 30-70 °C; (B) Cooling the solution thus obtained in a) up to a temperature equal to or below than room temperature; (C) Adding water up to a final volume ratio of ACN/H₂O of from 10:90 to 30:70; and (D)) Recovering the edoxaban tosylate monohydrate from the crystallization media wherein steps (B) and (C) are carried out in any order.

In a particular embodiment of the process, the volume ratio of ACN/H₂O in the recrystallization step, step e) of the process of the invention or in step (A) of the latter recrystallization process, is of from 50:50 to 35:65. In another particular embodiment of the process, the volume ratio of ACN/H₂O in the recrystallization step, step e) of the process of the invention or in step (A) of the latter recrystallization process, is 50:50. In another particular embodiment of the processes, in step e) or in the step (A) of the latter processes, the amount of ACN/H₂O is of from 4 to 7 volumes per weight of starting material calculated as edoxaban free base. In another particular embodiment of the processes, the amount of ACN/H₂O in step e) or in step (A) is 5-6 volumes per weight of the starting material calculated as edoxaban free base.

Generally, the starting edoxaban tosylate monohydrate is dissolved at a temperature of from 30 to 70 °C, preferably at a temperature of from 65 to 70 °C. Optionally, the process may comprise a seeding step to favor the crystallization of the product. A gradual cooling to room temperature is generally carried out. Preferably the cooling is continued until reaching a temperature of from 0 to 5 ° C.

In another particular embodiment of the processes, the amount of water added after the cooling step, in step g) of the process of the invention or in step (C) of the latter recrystallization process, is of from 4 to 10 volumes per weight of starting material calculated as edoxaban free base. In another particular embodiment of the processes, the amount of water added after the cooling step, in step g) of the process of the invention or in step (C) of the latter recrystallization process, is of from 5 to 8 volumes per weight of starting material calculated as edoxaban free base.

Generally, the additional water is added at a temperature comprised of from 50 to 0-5 °C. In a particular embodiment, the water is added at room temperature, in another particular embodiment, the water is added at 0-5 °C.

In a particular embodiment of these processes, the volume ratio of ACN/H₂O of the final mixture of step g) of the process of the invention or the step (C) of the purification process is of from 15:85 to 25:75. In another particular embodiment of these processes, the volume ratio of ACN/H₂O of the final mixture of step g) of the process of the invention or the step (C) of the purification process is 20:80.

In another particular embodiment of these processes, the amount of acetonitrile/water in the final crystallization media is 8-17 volumes of the mixture per weight of starting material calculated as edoxaban free base.

In another particular embodiment of these processes, the step of adding water is carried out at a temperature of from 0 to 5 °C.

In another preferred embodiment of these processes, the recovering step comprises filtering and drying the product thus obtained.

The combination of the one or more of the previous embodiments is also part of the invention.

The most adequate conditions for carrying out said process vary depending on the parameters considered by an expert in the art, such as, for example, the concentration of the reaction mixture, the temperature, and the like. These can be readily determined by said skilled person in the art with the help of the teachings of the examples given in this description.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

Analysis conditions for HPLC
Column: XBridge C18, 150 x 4.6 mm, 3.5 µm
Mobile phase: acetonitrile/phosphate buffer (pH = 3.0)
Temperature: 50 °C
Flow rate: 1.5 ml/min
Detection wavelength: 210 nm

Particle size distribution was determined using a Malvern Mastersizer 2000.

The powder X-ray diffractogram (PXRD) was acquired using a PANalytical X'Pert diffractometer using CuK-alpha radiation in transmission geometry.

The crystal form is compared to the crystal form of the reference standard which is Form I (see FIG. 1).

When seed crystals have been used in the following examples, they correspond to Form I.

### Example 1: Preparation of edoxaban tosylate monohydrate from edoxaban free base. Comparison of impurity A purification

### Example 1a: Preparation of edoxaban tosylate monohydrate in ACN/H₂O

N¹-(5-Chloropyridin-2-yl-)-N2-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide (1.6 g) (purity by HPLC: 98.1%a/a, Imp. A: 0.69%a/a) and p-toluenesulfonic acid monohydrate (0.58 g) were mixed with a mixture of ACN and water 1:1 (6.4 ml). The mixture was heated to 70 °C (0.47 °C/min) and after 30 minutes at this temperature a mixture of ACN and water 1:1 (1.6 ml) was added. The temperature was reduced to 50 °C (0.2 °C/min) and after 120 minutes at this temperature, to 2 °C (0.1 °C/min). Water (12 ml) was added at 2 °C in 120 minutes to the suspension. Then the reaction was stirred 60 minutes at 2 °C and filtered by vacuum. The crystals were washed with a solution of H₂O: ACN 8:2 and dried at 50 °C under reduced pressure. The outcome yield of the compound N¹-(5-Chloropyridin-2-yl-)-N²*-*((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl) ethanediamide p-toluenesulfonate monohydrate was 1.835 g (84.9%). Analysis by XRPD gave a diffractogram essentially the same as that represented by Figure 1. Purity by HPLC: 99.6%a/a, imp. A: 0.05%a/a.

### Comparative Example 1b: Preparation of edoxaban tosylate monohydrate in EtOH/H₂O

The methodology followed is the one of referential Example 3 of US8686189.

N¹-(5-Chloropyridin-2-yl-)-N²-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide (1.4 g) (purity by HPLC: 98.1 %a/a,: imp. A: 0.69%a/a) was mixed in 30% hydrous ethanol (6.74 ml) at 60 °C, and a solution (2.66 ml) of p-toluenesulfonic acid monohydrate (0.47 g) in 30% hydrous ethanol was added to the mixture. The mixture was stirred at 70 °C for 1 hour, and ethanol (1.4 ml) was added. Then the solution was gradually cooled to room temperature (0.25 °C/min). At room temperature, ethanol was added to the mixture (19.6 ml) followed by stirring for 16 hours. The mixture was cooled to 2 °C and stirred for 1 hour, and the formed crystals were recovered by vacuum filtration, washed with ethanol and dried at 50 °C under reduced pressure. The outcome yield of the compound N¹-(5-Chloropyridin-2-yl-)-N²-((1S,23,4S)-4-[(dimethylamino)carbonyl]-2-{[(5-methyl-9,5,6,7-tetrahydrothiazolo[5,4-c]pyridine-2-yl)carbonyl]amino} cyclohexyl)ethanediamide p-toluenesulfonate monohydrate was 1.632 g (86.3%). Analysis by XRPD gave a diffractogram essentially the same as that represented by Figure 1. Purity by HPLC: 99.7%a/a, imp. A: 0.08%a/a.

The comparison between Example 1 a and comparative Example 1 b shows that a high purification of the impurity N1-((1S,2R,4S)-2-amino-4-(dimethylcarbamoyl)cyclohexyl)-N2-(5-chloropyrid-2-yl)oxalamide (impurity A) is achieved by the process of the present invention when the starting material has a high content of impurity A: (0.69%a/a), even higher than in ethanol/water.

### Example 2: Preparation of edoxaban tosylate monohydrate from edoxaban free base with a high content of impurity A

A crude edoxaban free base with 2.86% w/w of impurity A in the form of a hydrochloride salt was used as starting material for Example 2a and comparative Example 2b.

### Example 2a: Preparation of edoxaban tosylate monohydrate in ACN/H₂O

N1-(5-Chloropyridin-2-yl-)-N2-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide (10.286 g with 2.86% w/w of impurity A hydrochloride salt) and p-toluenesulfonic acid monohydrate (3.64 g) were mixed in a mixture of ACN and water 1:1 (40 ml). Then the mixture was heated to 30 °C. After 3 hours, a mixture ACN/H₂O 1:1 (10 ml) was added and the mixture was cooled to 3 °C and maintained overnight. Water (75 ml) was added dropwise in 2 hours. The reaction was stirred 1 hour at 2 °C and filtered by vacuum. The crystals were washed with a solution of H₂O: ACN 8:2 and dried at 50 °C under reduced pressure. The outcome yield of the compound N¹-(5-Chloropyridin-2-yl-)-N²-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide p-toluenesulfonate monohydrate was 11.273 g (83.7%) HPLC: Imp. A: 0.15%a/a

### Comparative Example 2b: Preparation of edoxaban tosylate monohydrate in ethanol/water

To N1-(5-Chloropyridin-2-yl-)-N2-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide (10.286 g with 2.86% w/w of impurity A hydrochloride salt), 30% hydrous ethanol (48.2 ml) was added and the mixture was heated to 60 °C (0.6 °C/min). A solution (29 ml) of p-toluenesulfonic acid monohydrate (3.35 g) in 30% hydrous ethanol was added to the mixture. The temperature was increased to 70 °C and the solution was stirred for 1 hour and ethanol (3 ml) was added. Then, the solution was gradually cooled to room temperature (0.25 °C/min) and ethanol was added to the mixture (140 ml), followed by stirring for 16 hours. The mixture was cooled to 2 °C and stirred for 1 hour, and the formed crystals were recovered by vacuum filtration, washed with ethanol and dried at 50 °C under reduced pressure. The outcome yield of the compound N1-(5-Chloropyridin-2-yl-)-N2-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide p-toluenesulfonate monohydrate was 11.895 g (88.3%). HPLC: Imp. A: 0.22%a/a.

The comparison between Example 2a and comparative Example 2b confirms that a higher purification is achieved by the process of the present invention when the starting material has a very high content of impurity A (2.86%w/w).

### Example 3: Preparation of edoxaban tosylate monohydrate in ACN/H₂O at different conditions

Example 3a: N1-(5-Chloropyridin-2-yl-)-N2-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide (12 g) and p-toluenesulfonic acid monohydrate (4.37 g) were suspended in ACN (28.8 ml) and water (43.2 ml). The mixture was heated to 65-70 °C. The solution was filtered maintaining the temperature and the filter was washed using a mixture of 4.8 ml of ACN and 7.2 ml of water. The solution was cooled to 50 °C in 1 hour. The seed was added (120 mg) and conditions were maintained for 2 hours. The formed suspension was cooled to 40 °C in 30 minutes and conditions were kept again 2 hours. Finally, the suspension was cooled to 2 °C for at least 12 hours. Water was added (84 ml) at 2 °C in 120 minutes. Then the suspension was stirred at least 2 hours at 2 °C and filtered by vacuum. The crystals were washed twice with a cooled solution of H2O: ACN 8:2 and dried at 50 °C under reduced pressure. The outcome yield was 12.376 g (76.5%). HPLC: Imp. A: 0.013%a/a.
Example 3b: Example 3a was repeated but using less amount of initial solvent, 24 ml of acetonitrile and 24 ml of water, without performing the filtration, and cooling fast. Purity by HPLC: 99.6%a/a, imp. A: 0.03%a/a.
Example 3c: Example 3a was repeated but adding the acetonitrile/water as a mixture, heating at 30 °C, and fast cooling. Purity by HPLC: 99.66%a/a, imp. A: 0.01 %a/a.

### Example 4: Preparation of edoxaban tosylate monohydrate in ACN/H₂O

N1-(5-Chloropyridin-2-yl-)-N2-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide (2.75 g, purity by HPLC: 99.0%a/a, imp. A: 0.36%a/a) and of p-toluenesulfonic acid monohydrate (1.00 g) were suspended in ACN (5.5 ml) and water (5.5 ml). The mixture was heated to 70 °C. The solution was cooled to 50 °C (0.8 °C/min). Once the temperature was achieved, water was added (16.5 ml) in 1 hour. Then the suspension was cooled to 2 °C (0.2 °C/min). After 30 minutes the suspension was filtered by vacuum. The crystals were washed with a cooled solution of H₂O:ACN 8:2 and dried at 50 °C under reduced pressure. The outcome yield was 86.7%. Purity by HPLC: 99.6%a/a, imp. A: 0.03%a/a.

### Example 5: Preparation of edoxaban tosylate monohydrate in ACN/H₂O

N1-(5-Chloropyridin-2-yl-)-N2-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide (2g, purity by HPLC: 98.7%a/a, imp. A: 0.30%a/a) and of p-toluenesulfonic acid monohydrate (0.73 g) were suspended in ACN (4 ml) and water (4 ml). The mixture was heated to 65-70 °C. The solution was cooled to 20 °C (0.65 °C/min). Once the temperature was achieved, water was added (12 ml) in 1 hour. Then the suspension was cooled to 2 °C (0.2 °C/min). After 1h the suspension was filtered by vacuum. The crystals were washed with a cooled solution of H₂O: ACN 8:2 and dried at 50 °C under reduced pressure. The outcome yield was 84%. Purity by HPLC: 99.7%a/a, imp. A: 0.02%a/a.

### Example 6: Preparation of edoxaban tosylate monohydrate in ACN/H₂O. Study of impurity D purification (salt of edoxaban with an isomer of the p-toluensulfonic acid, orto isomer)

N1-(5-Chloropyridin-2-yl-)-N2-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide (12 g) and of p-toluenesulfonic acid monohydrate (4.37 g, the analysis of this batch reveals a 6.51% of isomer content by HPLC) were suspended in ACN (28.8 ml) and water (43.2 ml). The mixture was heated to 65-70 °C. The solution was filtered maintaining the temperature and the filter was washed using a mixture of 4.8 ml of ACN and 7.2 ml of water.The solution was cooled to 50 °C (0.33 °C/min). The seed was added (120 mg) and conditions were maintained for 2 hours. The formed suspension was cooled to 40 °C (0.33 °C/min) and conditions were kept again 2 hours. Finally, the suspension was cooled to 2 °C for at least 12 hours.Once the temperature was achieved, water was added (84 ml) at 2 °C in 120 minutes. Then the suspension was stirred at least 2 hours at 2 °C and filtered by vacuum. The crystals were washed twice with a cooled solution of H₂O:ACN 8:2 and dried at 50 °C under reduced pressure. The outcome yield was 12.92 g (80%). Purity by HPLC: 99.67%a/a, imp. D: 0.05%a/a.

### Example 7: Recrystallization of edoxaban tosylate monohydrate in ACN/H₂O

N1-(5-Chloropyridin-2-yl-)-N2-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide p-toluenesulfonate monohydrate (3.9 g) (Purity HPLC: 99.52%, imp. A: 0.05% a/a; imp. B: 0.16%a/a; imp. C: 0.11%a/a) was suspended in ACN (7.8 ml) and water (7.8 ml). The mixture was heated to 65 °C, and the temperature was kept for 10 minutes. Then the solution was cooled to 60 °C and kept for 30 min. Then, the solution was cooled to 20 °C (0.15 °C/min). Water (23.4 ml) was added at 20 °C in 120 minutes. Then the suspension was cooled to 2 °C (0.15 °C/min) and stirred overnight at 2 °C. Crystals deposited were collected by vacuum filtration, washed with a cooled mixture of ACN: H₂O 2:8, and dried at 50 °C under reduced pressure. The outcome yield was 3.37 g (86.4%). Analysis by XRPD gave a diffractogram essentially the same as that represented by Figure 1. The product corresponds to the same crystalline phase as the reference standard. Particle size distribution (PSD): d (0.9) = 30.685 µm. Purity HPLC: 99.85%, imp. A: nd; imp. B: 0.02%a/a; imp. C: 0.005%a/a.

### Example 8: Recrystallization of edoxaban tosylate monohydrate in ACN/H₂O

N1-(5-Chloropyridin-2-yl-)-N2-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide p-toluenesulfonate monohydrate (1.18 g mixture of product 1 and 2 in a ratio 70.7:29.3, purity HPLC product 1: 99.2%a/a, imp. A: nd; imp. B: 0.32%a/a; imp. C: 0.23%a/a; purity HPLC product 2: 99.2%a/a; imp. A: nd; imp. B: 0.33%a/a; imp. C: 0.16% a/a) was suspended in ACN (2.4 ml) and water (2.4 ml) at room temperature. The mixture was heated to 65 °C (0.5 °C/min) and after 30 minutes at this temperature, cooled to 50 °C (0.3 °C/min). After 1 hour at this temperature, the mixture was cooled to 2 °C (0.3 °C/min) and water (7 ml) was added at 2 °C in 60 minutes. Then the suspension was stirred for at least 2 hours at 2 °C and filtered by vacuum. The crystals were washed with a cooled solution of H₂O: ACN 8:2 and dried at 50 °C under reduced pressure. The outcome yield was 0.977 g (82.8%). Analysis by XRPD gave a diffractogram essentially the same as that represented by Figure 1. Purity HPLC: 99.7%a/a, imp. A: nd; imp.B: 0.02%a/a; imp. C: nd.

### Example 9: Effect of the recrystallization in the purification of impurity D

N1-(5-Chloropyridin-2-yl-)-N2-((1S,23,4S)-4-[(dimethylamino)carbonyl]-2-{[(5-methyl-9,5,6,7-tetrahydrothiazolo[5,4-c]pyridine-2-yl)carbonyl]amino}cyclohexyl)ethanediamide p-toluenesulfonate monohydrate (10 g, purity HPLC: 99.3%, imp. A: nd; imp. B: 0.03%a/a; imp. C: 0.03%a/a, imp. D: 0.30%a/a) was mixed with ACN (16.3 ml) and water (28.2 ml) at room temperature. The mixture was heated to 65-70 °C (1.5 °C/min). The solution was cooled to 50 °C (0.75 °C/min). The seed was added (70 mg) and conditions were maintained for 2 hours. The formed suspension was cooled to 40 °C (0.3 °C/min) and conditions were kept again for 2 hours. Finally, the suspension was cooled to 2 °C (0.05 °C/min) and water was added (37 ml) at 2 °C in 120 minutes. Then the suspension was stirred at least 2 hours at 2 °C and filtered by vacuum. The crystals were washed with a cooled solution of H2O:ACN 8:2 and dried at 50 °C under reduced pressure. The outcome yield was 8.778 g (87.8%). Purity HPLC: 99.82%, imp. A: nd; imp. B: nd; imp. C: nd; imp. D: nd).

### Citation list

### Patent Literature

- EP1405852
- US8686189B2
- EP2371830B1
- WO2015129603A1

## Claims

1. A preparation process of edoxaban tosylate monohydrate comprising:
a) Combining edoxaban free base, p-toluensulfonic acid in a mixture of acetonitrile/water in a volume ratio of from 70:30 to 30:70 and heating the mixture until reaching complete dissolution, wherein the mixture of acetonitrile/water is in such an amount that the edoxaban tosylate formed is dissolved at a temperature of from 30 to 70 °C;
b) Cooling the solution thus obtained in a) to a temperature equal to or below than room temperature;
c) Adding water up to a final volume ratio of acetonitrile/water of from 10:90 to 30:70; and
d) Recovering the edoxaban tosylate monohydrate thus obtained from the reaction media;
wherein steps b) and c) are carried out in any order.

2. The process according to claim 1, wherein the volume ratio of acetonitrile/water in step a) is of from 60:40 to 30:70.

3. The process according to any of the claims 1-2, wherein the amount of acetonitrile/water in step a) is of from 4 to 7 volumes of the mixture per weight of edoxaban free base starting material.

4. The process according to any of the claims 1-3, wherein the water in step c) is added at a temperature of from 0 to 5 °C.

5. The process according to any of the claims 1-4, wherein the amount of water added in step c) is of from 6 to 11 volumes per weight of edoxaban free base starting material.

6. The process according to any of the claims 1-5, wherein the volume ratio of acetonitrile/water of the final mixture of acetonitrile/water is of from 15:85 to 25:75.

7. The process according to any of the claims 1-6, wherein the total amount of acetonitrile/water in step c) is of from 10-18 volumes of a mixture of acetonitrile/water per weight of edoxaban free base starting material.

8. The preparation process according to any of the claims 1-7, which further comprises:
e) Dissolving the edoxaban tosylate monohydrate obtained in any of the claims 1-6 in a mixture of acetonitrile/water in a volume ratio acetonitrile/water from 60:40 to 30:70 at an appropriate temperature; wherein the mixture of acetonitrile/water is in such an amount that the edoxaban tosylate is dissolved at a temperature of from 30-70 °C;
f) Cooling the solution thus obtained in a) up to a temperature equal to or below than room temperature;
g) Adding water up to a final volume ratio of acetontrile/water of from 10:90 to 30:70; and
h) Recovering the edoxaban tosylate monohydrate from the crystallization media,
wherein steps f) and g) are carried out in any order.

9. A purification process of edoxaban tosylate monohydrate comprising:
(A) Dissolving edoxaban tosylate monohydrate in a mixture of acetonitrile/water in a volume ratio acetonitrile/water from 60:40 to 30: 70 at an appropriate temperature; wherein the mixture of acetonitrile/water is in such an amount that the edoxaban tosylate is dissolved at a temperature of from 30-70 °C;
(B) Cooling the solution thus obtained in a) up to a temperature equal to or below than room temperature;
(C) Adding water up to a final volume ratio of acetonitrile/water of from 10:90 to 30:70; and
(D) Recovering the edoxaban tosylate monohydrate from the crystallization media,
wherein steps (B) and (C) are carried out in any order.

10. The process according to any of the claims 8-9, wherein the initial ratio of acetonitrile/water is of from 50:50 to 35:65.

11. The process according to any of the claims 8-10, wherein the amount of acetonitrile/water in step e) or in the step (A) is of from 4 to 7 volumes of a mixture of acetonitrile/water per weight of starting material calculated as edoxaban free base.

12. The process according to any of the claims 8-11, wherein the amount of water added in step (g) or in step (C) is of from 4 to 10 volumes per weight of starting material calculated as edoxaban free base.

13. The process according to any of the claims 8-12, wherein the ratio of acetonitrile/water of the final mixture is of from 15:85 to 25:75.

14. The process according to any of the claims 8-13, wherein the water in step g) or in step (C) is added at a temperature of from 0 to 5 °C.

15. The process according to any of the claims 1-14, wherein the recovering step of the product comprises filtering and drying the product thus obtained.
